# EUROPÄISCHE PATENTANMELDUNG

(11) **EP 4 591 840 A1**
(43) Veröffentlichungstag der Anmeldung: **30.07.2025**
(21) Anmeldenummer: 24153635.8
(22) Anmeldetag: 24.01.2024
(51) Int. Cl.: A61F 5/01

(54) **HANDORTHESE**

(71) Anmelder: TP-Ortho GmbH, 53474 Bad Neuenahr-Ahrweiler (DE); Masalo KG, 21337 Lüneburg (DE)
(72) Erfinder: Pütz, Thomas, 53498 Bad Breisig (DE)
(74) Vertreter: Pelster Behrends Patentanwälte PartG mbB

(57) **Zusammenfassung**

Die Erfindung betrifft eine Handorthese (10) mit zumindest einer Fingeraufnahme (12), welche dazu eingerichtet ist, zumindest einen Finger (102) einer Hand (100) zumindest bereichsweise aufzunehmen, insbesondere zu umschließen.

## Beschreibung

Die Erfindung betrifft eine Handorthese nach dem Oberbegriff des Patentanspruchs 1, eine Orthesenanordnung nach dem Oberbegriff des Patentanspruchs 12 und ein Verfahren zum Einstellen nach dem Oberbegriff des Patentanspruchs 14.

Orthesen sind aus dem Bereich der Medizin bekannte medizinische Hilfsmittel, welche als äußerlich angelegte Geräte zur Beeinflussung der strukturellen und/oder funktionellen Eigenschaften des neuromuskulären und/oder skelettalen Systems, beispielsweise zur Unterstützung der Therapie und/oder der Heilung nach einer Verletzung und/oder einer Operation an einem Körperteil, beispielsweise einer Hand, eingesetzt werden. So werden Handorthesen beispielsweise bei der Therapie des Karpaltunnelsyndroms eingesetzt, um die Erfolgschancen der Therapie zu erhöhen und die Funktionsfähigkeit des Handgelenks schnellstmöglich wiederherzustellen und/oder zu erhalten und/oder die Muskulatur der Hand und/oder des Unterarms während der Therapie zu stärken.

Die aus dem Stand der Technik bekannten Handorthesen sind darauf ausgelegt, den Bereich der Hand und/oder des Unterarms, insbesondere die Finger und/oder das Handgelenk, während einer Therapie vollständig oder zumindest teilweise ruhigzustellen. Eine vollständige oder zumindest teilweise Ruhigstellung der Hand kann allerdings dazu führen, dass die Hand in einer für die Therapie ungünstigen und/oder in einer unnatürlichen Stellung ruhiggestellt wird, wodurch sich die Therapiedauer verlängern und/oder der Therapieerfolg vermindern kann und/oder sogar zusätzliche negative gesundheitliche Folgen entstehen können, beispielsweise eine Verschlimmerung der Symptome des Karpaltunnelsyndroms. Insbesondere kann die Ruhigstellung der Hand zu einer Schwächung der Hand und/oder Unterarmmuskulatur führen.

Des Weiteren sind die aus dem Stand der Technik bekannten Handorthesen nicht dazu geeignet, eine sogenannte Fallhand, welche beispielsweise die Folge eines Traumas oder eines Schlaganfalls sein kann, zu behandeln oder bei der therapeutischen Behandlung anderer Erkrankungen oder Verletzungen im Handbereich eine Fallhand zu vermeiden. Das Phänomen der Fallhand beschreibt eine Handposition und/oder eine Handstellung, bei welcher das Handgelenk und/oder ein oder mehrere Finger nicht mehr ausreichend durchgestreckt werden können. Diese als Fallhand bezeichnete längerfristig vorliegende gewinkelte Stellung eines oder mehrerer Finger und/oder des Handgelenks kann, wie bereits beschrieben, den Therapieerfolg schmälern, die Therapiedauer verlängern und/oder sogar zu einer Verschlimmerung der Symptomatik, im schlimmsten Fall sogar zu einer Nervenschädigung, führen.

Die der Erfindung zugrunde liegende Aufgabe besteht somit darin, eine verbesserte Handorthese für einen erhöhten Therapieerfolg und/oder eine verkürzte Therapiedauer bereitzustellen.

Die Aufgabe wird gelöst durch eine Handorthese der eingangs genannten Art, wobei die Handorthese eine Verstelleinrichtung umfasst, welche dazu eingerichtet ist, eine auf die Fingeraufnahme wirkende Korrekturkraft zu erzeugen, durch welche der zumindest eine Finger in eine korrigierte Stellung verbringbar ist.

Dadurch, dass die Handorthese eine Verstelleinrichtung umfasst, kann die Stellung einer Hand, an welcher die erfindungsgemäße Handorthese angelegt ist, mittels einer von der Verstelleinrichtung erzeugten Korrekturkraft korrigiert werden, wobei durch die Korrekturkraft und das Verbringen der Hand in die korrigierte Stellung insbesondere verhindert, dass die Hand durch eine vollständige Ruhigstellung in einer die Therapiedauer und/oder den Therapieerfolg negativ beeinflussenden Position und/oder Stellung, ruhiggestellt wird. Durch das Erzeugen einer Korrekturkraft kann die Hand außerdem in eine Stellung verbracht werden, welche das Auftreten einer Fallhand verhindert, indem die Finger und/oder das Handgelenk der Hand durch einen Gegenzug und eine daraus resultierende kontrollierte Bewegung des Handgelenks und/oder eines oder mehrerer Finger durchgestreckt werden, sodass beispielsweise eine Reizung und/oder Belastung im Bereich des Karpaltunnels reduziert und/oder vermieden wird und/oder ein Abbau der Unterarmmuskulatur reduziert und/oder vermieden wird, sodass der Therapieerfolg, beispielsweise nach einer Operation und/oder nach einer Verletzung, maximiert und die Therapiedauer minimiert werden kann und die Heilung somit optimal unterstützt wird. Durch die Verstelleinrichtung der Handorthese und die dadurch erzeugbare kontrollierte Bewegung eines oder mehrerer Finger oder der ganzen Hand kann somit statt einer vollständigen oder teilweisen Ruhigstellung der Hand eine Dynamisierung der Handstellung und/oder Fingerstellung realisiert werden.

Vorzugsweise ist die Verstelleinrichtung der Handorthese dazu eingerichtet, mittels der erzeugten Korrekturkraft die Hand und/oder das Handgelenk alternativ oder zusätzlich zu dem zumindest einen Finger in eine korrigierte Stellung zu bringen. Die zumindest eine Fingeraufnahme der Handorthese ist vorzugsweise als Fingerring, insbesondere als elastischer Fingerring, ausgebildet. Die zumindest eine Fingeraufnahme kann beispielsweise als Schlaufe, insbesondere als ringförmige Schlaufe, ausgebildet sein, welche den Finger ganz oder zumindest teilweise entlang seines Umfangs umschließt und/oder flächig eng an dem jeweiligen Finger anliegt. Vorzugsweise ist die Fingeraufnahme dazu eingerichtet, die mittels der Verstelleinrichtung erzeugte Korrekturkraft auf den jeweiligen Finger zu übertragen. Insbesondere ist die Fingeraufnahme dazu eingerichtet, infolge der auf die Fingeraufnahme wirkenden Korrekturkraft eine Zug- und/oder eine Druckkraft auf den jeweiligen Finger auszuüben und den jeweiligen Finger auf diese Weise in eine korrigierte Stellung zu drücken und/oder zu ziehen. Vorzugsweise wird durch die Korrekturkraft eine Korrekturbewegung des jeweiligen Fingers in die korrigierte Stellung erzeugt. Alternativ oder zusätzlich kann die Korrekturkraft zu einer Korrekturbewegung der gesamten Hand, insbesondere des Handgelenks, führen, um die Hand und/oder das Handgelenk in eine korrigierte Stellung zu verbringen.

Vorzugsweise umfasst die Handorthese mehrere Fingeraufnahmen, insbesondere zwei, drei, vier oder fünf Fingeraufnahmen, welche jeweils einem Finger zugeordnet sind und/oder jeweils einen Finger umschließen, um die mittels der Verstelleinrichtung erzeugte Korrekturkraft auf mehrere Finger gleichzeitig ausüben zu können, sodass mehrere Finger und/oder die gesamte Hand und/oder das Handgelenk in eine beabsichtigte korrigierte Stellung verbringbar sind. Auf diese Weise wird es ermöglicht, zumindest einen Finger und/oder zumindest ein Fingergelenk und/oder zumindest ein Handgelenk und/oder zumindest eine Hand eines Patienten mittels der Handorthese so zu bewegen und/oder zu stabilisieren und/oder ruhigzustellen und/oder so in eine beabsichtigte korrigierte Stellung zu verbringen, dass eine mittels der Handorthese durchgeführte Therapie der jeweiligen Hand eine höhere Erfolgswahrscheinlichkeit hat und/oder die Therapiedauer verkürzt werden kann, insbesondere durch das Verhindern einer Fallhand. Vorzugsweise wird die Hand und/oder das Handgelenk und/oder der Finger mittels der Korrekturkraft in eine Neutralstellung gebracht und/oder in einer Neutralstellung gehalten, sodass eine Fallhand vermieden werden kann. Vorzugsweise wird mittels der Handorthese, insbesondere durch das Erzeugen der Korrekturkraft mittels der Verstelleinrichtung, eine Entlastung der Hand und/oder des Fingers und/oder des Handgelenks bewirkt.

Die Handorthese kann vollständig aus einem Material, beispielsweise aus einem elastischen oder aus einem nicht elastischen Material ausgebildet sein. Zudem kann die Handorthese aus unterschiedlichen Materialien, insbesondere einem Materialmix, ausgebildet sein, wobei die Handorthese, beispielsweise verschiedene Bereiche der Handorthese, aus elastischen und nicht elastischen Materialien zusammengesetzt sein können. Elastische Materialien sind dadurch gekennzeichnet, dass sie unter Krafteinwirkung eine reversible Dehnung erfahren. Nicht elastische Materialien sind dadurch gekennzeichnet, dass sie durch ihre Nichtelastizität, insbesondere durch eine höhere Festigkeit und/oder eine höhere Zug- und/oder Druckkraftstabilität, keine elastische Verformbarkeit aufweisen und sich unter Krafteinwirkung nicht oder nur geringfügig dehnen. Die zumindest eine Fingeraufnahme der Handorthese ist vorzugsweise aus einem elastischen Material ausgebildet und/oder umfasst zumindest bereichsweise ein elastisches Material. Beispielsweise kann die Fingeraufnahme aus einem Silikon, Neopren, Elastomer und/oder aus Kautschuk ausgebildet sein.

In einer bevorzugten Ausführungsform der erfindungsgemäßen Handorthese umfasst die Handorthese zumindest ein Verbindungselement, welches die Verstelleinrichtung mit der zumindest einen Fingeraufnahme verbindet und dazu eingerichtet ist, die mittels der Verstelleinrichtung erzeugte Korrekturkraft auf die Fingeraufnahme zu übertragen. Vorzugsweise erstreckt sich das Verbindungselement entlang des Handrückens der Hand und/oder kontaktiert den Handrücken zumindest bereichsweise. Insbesondere liegt das Verbindungselement flächig und eng an dem Handrücken der Hand an. Außerdem kann sich das Verbindungselement zumindest abschnittsweise entlang eines oder mehrerer Finger der Hand erstrecken und/oder einen oder mehrere Finger zumindest bereichsweise kontaktieren und/oder zumindest bereichsweise eng an einem oder mehreren Fingern anliegen.

Vorzugsweise ist das Verbindungselement an einem ersten Ende mit der Verstelleinrichtung verbunden und/oder an der Verstelleinrichtung befestigt. Dadurch, dass das Verbindungselement mit einem Ende mit der Verstelleinrichtung verbunden und/oder an der Verstelleinrichtung befestigt ist, kann die mittels der Verstelleinrichtung erzeugte Korrekturkraft auf das Verbindungselement übertragen werden, insbesondere kann eine Zugkraft auf das Verbindungselement übertragen werden. Vorzugsweise weist das Verbindungselement ein zweites Ende auf, welches mit der zumindest einen Fingeraufnahme verbunden und/oder an der zumindest einen Fingeraufnahme befestigt ist. Weist die Handorthese mehrere Fingeraufnahmen auf, weist das Verbindungselement vorzugsweise eine zu der Anzahl der Fingeraufnahmen korrespondierende Anzahl an Enden auf, sodass das Verbindungselement mit jeder der mehreren Fingeraufnahmen verbunden und/oder an jeder der weiteren Fingeraufnahmen befestigt ist. Dadurch, dass das Verbindungselement mit der einen oder den mehreren Fingeraufnahmen verbunden und/oder an der einen oder den mehreren Fingeraufnahmen befestigt ist, kann die mittels der Verstelleinrichtung erzeugte Korrekturkraft, welche insbesondere über das erste Ende des Verbindungselements auf das Verbindungselement übertragen wird, auf die eine oder die mehreren Fingeraufnahmen übertragen werden. Insbesondere wird durch das Verbindungselement und die mit der einen oder den mehreren Fingeraufnahmen verbundenen Enden des Verbindungselements eine Zugkraft auf die Fingeraufnahmen übertragen, durch welche der eine oder die mehreren Finger in eine beabsichtigte korrigierte Stellung verbracht werden können.

Das Verbindungselement kann zumindest abschnittsweise bandförmig ausgebildet sein. Insbesondere kann das Verbindungselement zwischen seinem mit der Verstelleinrichtung verbundenen Ende und dem einen oder den mehreren mit der einen oder den mehreren Fingeraufnahmen verbundenen Enden eine sich über die Länge des Verbindungselements verändernde Breite aufweisen. Zudem kann das Verbindungselement schlitzförmige Ausnehmungen umfassen, welche das Verbindungselement in mit dem einen oder den mehreren Fingern der Hand korrespondierende, insbesondere bandförmige, Abschnitte unterteilt. Vorzugsweise verlaufen die Abschnitte des Verbindungselements entlang und/oder parallel zu der Längserstreckung des jeweiligen Fingers.

In einer anderen bevorzugten Ausführungsform der erfindungsgemäßen Handorthese ist das Verbindungselement aus einem elastischen und/oder flexiblen Material ausgebildet und/oder umfasst das Verbindungselement ein elastisches und/oder flexibles Material. Alternativ oder zusätzlich ist das Verbindungselement aus einem nicht-elastischen und/oder nicht-flexiblen Material ausgebildet und/oder umfasst das Verbindungselement ein nicht-elastisches und/oder ein nicht-flexibles Material. Vorzugsweise sind das Verbindungselement und die zumindest eine Fingeraufnahme Bestandteil eines gemeinsamen integralen Körpers. Vorzugsweise umfasst die Handorthese zumindest ein elastisches Verbindungselement, welches beispielsweise gurt- oder bandförmig ausgebildet ist und die zumindest eine Fingeraufnahme mit der Verstelleinrichtung so verbindet, dass eine von der Verstelleinrichtung erzeugte Kraft, insbesondere eine Zugkraft, durch Ziehen an dem elastischen Verbindungselement unter elastischer Verformung, insbesondere unter Dehnung des Verbindungselements, auf die zumindest eine Fingeraufnahme überträgt.

Das Verbindungselement ist vorzugsweise zumindest bereichsweise und/oder abschnittsweise aus einem oder mehreren elastischen Materialien ausgebildet. Insbesondere kann das Verbindungselement aus Silikon, Neopren, aus einem Elastomer und/oder aus Kautschuk bestehen und/oder zumindest bereichsweise und/oder abschnittsweise Silikon, Neopren, ein Elastomer und/oder Kautschuk umfassen. Ist das Verbindungselement aus einem elastischen Material ausgebildet und/oder umfasst das Verbindungselement ein elastisches Material, ist das Verbindungselement unter Krafteinwirkung, insbesondere unter Einwirkung der Korrekturkraft, elastisch verformbar und/oder elastisch dehnbar, wobei durch die durch die elastische Verformbarkeit durch die Zugkraft hervorgerufene Zugspannung in dem Verbindungselement die Korrekturkraft von der Verstelleinrichtung auf die zumindest eine Fingeraufnahme überträgt. Durch die elastische Verformbarkeit und/oder Dehnbarkeit des Verbindungselements kann das Verbindungselement insbesondere eine Federwirkung erzeugen.

Das Verbindungselement kann alternativ oder zusätzlich aus einem Material ausgebildet sein und/oder ein Material zumindest bereichsweise und/oder abschnittsweise umfassen, welches eine geringere Elastizität als das vorstehend beschriebene elastische Material aufweist. Insbesondere kann das Verbindungselement aus einem nicht-dehnbaren und/oder nicht-elastischen und/oder festen, insbesondere zugfesten und/oder formstabilen, Material ausgebildet sein und/oder dieses umfassen. Ein nicht-dehnbares und/oder nicht-elastisches Material und/oder ein weniger elastisches und/oder weniger dehnbares Material weist unter Einwirkung einer äußeren Kraft, insbesondere unter Einwirkung der Korrekturkraft, keine oder zumindest eine reduzierte elastische Verformbarkeit auf und ist somit formstabiler.

Das Verbindungselement und die zumindest eine Fingeraufnahme können integraler Bestandteil eines einstückigen Körpers sein. Insbesondere können das Verbindungselement und die zumindest eine Fingeraufnahme gemeinsam eine Handaufnahme ausbilden, welche die Hand zumindest bereichsweise kontaktiert und/oder die Hand zumindest bereichsweise umschließt. Eine Handaufnahme ist vorzugsweise dazu eingerichtet, die Hand eines Patienten zumindest bereichsweise aufzunehmen, insbesondere kann die Handaufnahme einen Teil der Hand, insbesondere den Handrücken zumindest bereichsweise bedecken und/oder kontaktieren, insbesondere flächig kontaktieren.

Vorzugsweise ist das Verbindungselement aus dem gleichen Material und/oder Materialmix ausgebildet wie die zumindest eine Fingeraufnahme. Das Verbindungselement und die zumindest eine Fingeraufnahme können alternativ zwei separate und miteinander verbundene und/oder aneinander befestigte Bestandteile der Handorthese sein, welche auch aus unterschiedlichen Materialien und/oder Materialmixen ausgebildet sein können. Somit kann die Charakteristik der Korrekturkraftübertragung von der Verstelleinrichtung auf den zumindest einen Finger durch die Materialwahl und/oder die Materialkombination des Verbindungselements und/oder der zumindest einen Fingeraufnahme individuell verändert werden, insbesondere durch die Wahl der Elastizität des jeweiligen Materials und/oder die Anordnung, in welchem Bereich ein jeweiliges Material verwendet wird.

In einer weiteren bevorzugten Ausführungsform der erfindungsgemäßen Handorthese ist die Verstelleinrichtung als mechanische Verstelleinrichtung ausgebildet, wobei die Korrekturkraft vorzugsweise mittels eines Einstellglieds der Verstelleinrichtung erzeugbar und/oder verstellbar ist und/oder wobei das Einstellglied der Verstelleinrichtung zum Fixieren einer beabsichtigten Korrekturkraft in einer gewünschten Stellung festsetzbar ist. Vorzugsweise ist die Korrekturkraft individuell und/oder stufenlos mittels des Einstellglieds verstellbar. Die Verstelleinrichtung kann beispielsweise als Ratschenverschluss und/oder als Schnellverschlusssystem und/oder als Drehverschluss und/oder als Drehratschenverschluss ausgebildet sein. Insbesondere kann das Einstellglied als ein beispielsweise von Schuhen bekannter BOA-Verschluss ausgebildet sein.

Die Verstelleinrichtung kann als Seilzug ausgebildet sein und/oder einen Seilzug umfassen. Durch die Verstelleinrichtung der Handorthese ist die auf den zumindest einen Finger und/oder auf die Hand wirkende Korrekturkraft schnell, präzise und einfach individuell einstellbar, sodass eine gewünschte Korrekturkraft für die jeweilige individuelle Therapie für einen Patienten eingestellt werden kann. Vorzugsweise ist die mittels der Verstelleinrichtung eingestellte Korrekturkraft und/oder die daraus resultierende korrigierte Stellung in der gewünschten Stellung und/oder Position verriegelbar und/oder fixierbar, sodass eine gewünschte Korrekturkraft und/oder eine daraus resultierende gewünschte korrigierte Stellung von der Handorthese langfristig beibehalten wird. Vorzugsweise ist das Einstellglied der Verstelleinrichtung zum Lösen und/oder zum Verriegeln der gewünschten Korrekturkraft und/oder der gewünschten korrigierten Stellung verriegelbar und entriegelbar, insbesondere mittels einer Entriegelungsbewegung und/oder Verriegelungsbewegung des Einstellglieds.

Vorzugsweise umfasst die Verstelleinrichtung, insbesondere das Einstellglied der Verstelleinrichtung, eine Rasterung und/oder eine Verzahnung, durch welche beim Erzeugen und/oder beim Einstellen der Korrekturkraft ein haptisches und/oder akustisches Feedback ausgegeben wird und/oder mittels welcher die Korrekturkraft und/oder die korrigierte Stellung bei einem beabsichtigten Wert fixierbar ist.

Alternativ oder zusätzlich kann die Verstelleinrichtung, insbesondere das Einstellglied der Verstelleinrichtung, elektrisch ausgebildet sein, sodass die Korrekturkraft und/oder die fixierte Stellung, insbesondere das Einstellen und/oder Erzeugen der Korrekturkraft und/oder der korrigierten Stellung elektrisch herbeigeführt und/oder elektrisch unterstützt werden kann. Eine elektrische Verstelleinrichtung kann beispielsweise einen Schalter und/oder einen Regler umfassen, mittels welchem das Erzeugen der Korrekturkraft und/oder das Verbringen in die korrigierte Stellung initiiert werden kann. Eine elektrische Verstelleinrichtung kann beispielsweise einen Elektromotor und/oder zur Energieversorgung der Verstelleinrichtung einen Akku und/oder eine Batterie umfassen.

In einer Weiterbildung der erfindungsgemäßen Handorthese ist das Einstellglied als drehbares Einstellglied, insbesondere als Drehverschluss, ausgebildet. Vorzugsweise ist die Korrekturkraft durch eine Drehbewegung des Einstellglieds in einer Drehrichtung erzeugbar und/oder einstellbar, insbesondere erhöhbar und/oder reduzierbar. Vorzugsweise ist das Einstellglied als Drehknopf ausgebildet. Das drehbare Einstellglied kann zum Fixieren der aktuellen Korrekturkraft und/oder der aktuell eingestellten korrigierten Stellung vorzugsweise in einer gewünschten Drehstellung verriegelt werden, um eine weitere unbeabsichtigte Drehbewegung des Einstellglieds zum Verändern der Korrekturkraft zu blockieren. Zudem kann eine Verriegelung des Einstellglieds zum Entriegeln des Einstellglieds und somit zum Entriegeln der Korrekturkraft und/oder der eingestellten korrigierten Stellung entriegelt werden. Zum Verriegeln und/oder Entriegeln kann das Einstellglied beispielsweise senkrecht zur Drehebene herausziehbar und/oder hineindrückbar sein.

Vorzugsweise weist das Einstellglied eine Beschriftung und/oder eine Skala auf, über welche ein aktuell eingestellter Korrekturkraftwert und/oder die korrekte Drehrichtung zum Einstellen der Korrekturkraft ablesbar ist. Das Einstellglied kann zudem eine strukturierte Oberfläche, insbesondere eine Riffelung aufweisen, durch welche das Drehen des Einstellglieds, beispielsweise durch einen Patienten, erleichtert wird. Das Einstellglied der Verstelleinrichtung kann alternativ als lineares Einstellglied, insbesondere als linear verstellbares Einstellglied, ausgebildet sein, wobei die Korrekturkraft und/oder die korrigierte Stellung durch eine Linearbewegung des Einstellglieds verstellbar ist. Ein linear verstellbares Einstellglied kann beispielsweise ein in einer Schiene geführter und in der Schiene fixierbarer Einstellkörper sein.

Es ist zudem eine erfindungsgemäße Handorthese bevorzugt, bei welcher die Verstelleinrichtung ein Übertragungsglied zum Übertragen der Korrekturkraft von dem Einstellglied der Verstelleinrichtung auf die Fingeraufnahme, insbesondere über das Verbindungselement, umfasst. Vorzugsweise ist das Übertragungsglied mit dem Einstellglied der Verstelleinrichtung verbunden und/oder an dem Einstellglied der Verstelleinrichtung befestigt. Insbesondere kann das Übertragungsglied mit einem Seilzug des Einstellglieds verbunden und/oder an dem Seilzug des Einstellglieds befestigt sein. Ist das Einstellglied als drehbares Einstellglied ausgebildet, führt eine Drehbewegung des Einstellglieds vorzugsweise zu einer Linearbewegung und/oder einer Lineardehnung des Übertragungsglieds, insbesondere zu einer linearen Kraftübertragung, insbesondere einer Zugkraft, von dem Einstellglied auf das Verbindungselement und/oder die zumindest eine Fingeraufnahme der Handorthese. Ein Drehen des Einstellglieds übt somit eine Kraft auf das Übertragungsglied auf, wobei die auf das Übertragungsglied ausgeübte Kraft insbesondere der Korrekturkraft entsprechen kann, wobei die Kraft des Übertragungsglieds auf das Verbindungselement und von dem Verbindungselement auf die zumindest eine Fingeraufnahme übertragen wird, sodass die mittels der Drehbewegung des Einstellglieds erzeugte Drehbewegung und die daraus resultierende Kraft über das Übertragungsglied, das Verbindungselement und die Fingeraufnahme auf den Finger und/oder die Hand zum Herbeiführen der korrigierten Stellung übertragen wird.

Ist das Einstellglied als lineares Einstellglied, beispielsweise als Linearverschlusssystem, als Spanngurtverschluss und/oder Zurrgurtverschluss mit Ratsche, ausgebildet, kann eine lineare Bewegung des Einstellglieds auf das Übertragungsglied, insbesondere eine lineare Bewegung des Übertragungsglieds und/oder eine linear auf das Übertragungsglied wirkende Kraft, sein, wobei die lineare Bewegung und/oder die lineare Kraft des Übertragungsglieds über das Verbindungselement auf die zumindest eine Fingeraufnahme übertragen wird.

Vorzugsweise wird über die Bewegung des Übertragungsglieds eine Zugkraft auf das Verbindungselement ausgeübt. Dadurch wird vorzugsweise eine Zugkraft auf die Fingeraufnahme ausgeübt. Vorzugsweise sind das Übertragungsglied und die Fingeraufnahme und/oder das Übertragungsglied und das Verbindungselement miteinander verbunden und/oder aneinander befestigt. Alternativ oder zusätzlich kann die Verstelleinrichtung eine Feder, insbesondere eine Spiralfeder umfassen, welche eine Zugkraft, insbesondere die Korrekturkraft, erzeugt und/oder eine Kraftübertragung auf die zumindest eine Fingeraufnahme realisiert.

Es ist weiterhin eine erfindungsgemäße Handorthese vorteilhaft, bei welcher das Übertragungsglied aus einem elastischen und/oder flexiblen Material ausgebildet ist und/oder ein elastisches und/oder flexibles Material umfasst. Alternativ oder zusätzlich ist das Übertragungsglied aus einem nicht elastischen und/oder nicht flexiblen Material ausgebildet und/oder umfasst ein nicht elastisches oder nicht flexibles Material. Vorzugsweise ist das Übertragungsglied bandförmig ausgebildet und/oder mit dem Verbindungselement verbunden. Insbesondere kann das Übertragungsglied mit der Handaufnahme, welche sich aus der Fingeraufnahme und dem Verbindungselement zusammensetzt, verbunden und/oder an diesem befestigt sein. Vorzugsweise ist das Übertragungsglied gurtförmig, seilförmig und/oder bandförmig ausgebildet. Die zumindest eine Fingeraufnahme, das Verbindungselement, die Handaufnahme und/oder das Übertragungsglied können integraler Bestandteil eines einstückigen Körpers insbesondere eines einstückigen elastischen Körpers aus einem elastischen Material sein. Das Übertragungsglied ist vorzugsweise als Gummiband ausgebildet. Zudem kann das Gummiband aus einem anderen Elastomer, aus Neopren, aus Silikon und/oder aus Kautschuk ausgebildet sein. Das Übertragungsglied kann aus dem gleichen Material oder dem gleichen Materialmix ausgebildet sein wie das Verbindungselement und/oder die zumindest eine Fingeraufnahme.

In einer weiteren bevorzugten Ausführungsform der erfindungsgemäßen Handorthese umfasst die Handorthese einen Korrekturindikator, über welchen erfassbar ist, ob die mittels der Verstelleinrichtung eingestellte Korrekturkraft und/oder die daraus resultierende korrigierte Stellung des zumindest einen Fingers in einem beabsichtigten Korrekturbereich liegen. Vorzugsweise ist über den Korrekturindikator eine aktuell eingestellte Korrekturkraft und/oder eine aktuell eingestellte korrigierte Stellung ablesbar, beispielsweise durch Darstellung eines Zahlenwerts, beispielsweise einer Kraft in Newton, und/oder durch Darstellung eines oder mehrerer Signale, welche auf eine Korrekturkraft und/oder eine aktuell eingestellte korrigierte Stellung hinweisen. Insbesondere kann der Korrekturindikator als Positionsanzeige ausgebildet sein, welche anzeigt, ob eine aktuell vorliegende Finger- und/oder Handposition mit einer beabsichtigten Finger- und Handposition übereinstimmt. Zudem kann der Korrekturindikator dazu eingerichtet sein, eine Warnung und/oder einen Warnhinweis auszugeben, sofern eine aktuelle Hand- und/oder Fingerposition nicht der beabsichtigten korrigierten Stellung entspricht und/oder sobald sich die Hand- und/oder Fingerposition aus einem beabsichtigten Korrekturbereich herausbewegt. Auf diese Weise ist mittels des Korrekturindikators ablesbar und/oder kontrollierbar, ob eine beabsichtigte und/oder korrekte Korrekturkraft und/oder korrigierte Stellung vorliegt und/oder wann beim Erzeugen und/oder Verändern der Korrekturkraft die beabsichtigte korrigierte Stellung erreicht ist.

Vorzugsweise erstreckt sich das Übertragungsglied durch den Korrekturindikator hindurch, wobei eine Bewegung und/oder elastische Dehnung des Übertragungsglieds, aus welcher die Korrekturkraft und/oder die korrigierte Stellung resultiert, zu einer Zustandsänderung des Korrekturindikators führen kann. Mittels des Korrekturindikators wird es vorzugsweise ermöglicht, insbesondere zu Beginn einer Therapie nach erstmaligem Anlegen der Handorthese, die Handorthese auf den zu der Therapie passenden Wert einer Korrekturkraft einzustellen und/oder in eine beabsichtigte zu der Therapie passende korrigierte Stellung zu verbringen, um im weiteren Verlauf der Therapie frühzeitig zu erkennen, sobald eine Veränderung der Korrekturkraft und/oder der korrigierten Stellung dazu führt, dass sich die Finger- und/oder Handposition aus dem beabsichtigten Korrekturbereich herauszubewegen droht. Folglich kann mittels des Korrekturindikators frühzeitig erkannt werden, wenn sich die Handposition in die sogenannte Fallhandposition bewegt. Durch das Bewegen der Hand- und/oder Fingerposition in die Fallhand üben die Hand und/oder die Finger eine Gegenkraft auf die Verstelleinrichtung und/oder das Übertragungsglied und/oder das Verbindungselement und/oder die Fingeraufnahme aus, wobei die Gegenkraft dazu führen kann, dass der Korrekturindikator eine Warnung anzeigt, dass sich die Hand nicht mehr in der beabsichtigten Stellung befindet.

In einer anderen bevorzugten Ausführungsform der erfindungsgemäßen Handorthese ist der Korrekturindikator dazu eingerichtet, ein die eingestellte Korrekturkraft und/oder die daraus resultierende korrigierte Stellung betreffendes optisches und/oder haptisches und/oder akustisches Signal zu erzeugen, wobei der Korrekturindikator vorzugsweise Bestandteil der Verstelleinrichtung ist. Insbesondere kann der Korrekturindikator die eingestellte Korrekturkraft und/oder die daraus resultierende korrigierte Stellung mittels Kontrollfarben anzeigen. Ist beispielsweise eine beabsichtigte Korrekturkraft eingestellt und/oder ist aktuell die beabsichtigte korrigierte Stellung eingestellt, kann der Korrekturindikator beispielsweise durch Anzeigen eines grünen Kontrollbereichs signalisieren, dass die Handorthese, insbesondere die Verstelleinrichtung, korrekt eingestellt ist. Ist die eingestellte Korrekturkraft außerhalb eines vorgegebenen Korrekturkraftbereichs und/oder ist die aktuelle korrigierte Stellung außerhalb eines vorgegebenen Stellungsbereichs und/oder liegt aktuell eine Veränderung der Korrekturkraft und/oder der Stellung vor, welche dazu führen könnte, dass die Hand in die Fallhand rutscht, kann beispielsweise ein roter Kontrollbereich Handlungsbedarf anzeigen. Auf diese Weise kann schnell und einfach durch Anzeige eines roten oder grünen Signals des Korrekturindikators abgelesen werden, ob die Handorthese korrekt eingestellt ist.

Alternativ oder zusätzlich kann der Korrekturindikator ein rotes und/oder grünes Signallämpchen zur Kontrolle der Handposition umfassen. Alternativ oder zusätzlich kann der Korrekturindikator einen zu der aktuell eingestellten Korrekturkraft und/oder korrigierten Stellung korrespondierenden Signalton ausgeben. Zudem kann beim Einstellen der Korrekturkraft und/oder beim Einstellen der korrigierten Stellung ein haptisches Feedback von dem Korrekturindikator, beispielsweise ein spürbares Knacken, ausgegeben werden, welches auf das Erreichen der beabsichtigten Korrekturkraft und/oder der beabsichtigten korrigierten Stellung hindeutet.

In einer Weiterbildung der erfindungsgemäßen Handorthese umfasst die Handorthese eine Unterarmaufnahme, welche dazu eingerichtet ist, einen Unterarm zumindest bereichsweise aufzunehmen, insbesondere zu umschließen, wobei die Verstelleinrichtung vorzugsweise an der Unterarmaufnahme angeordnet ist und/oder die Unterarmaufnahme mit der Fingeraufnahme und/oder mit dem Verbindungselement verbunden ist. Die Unterarmaufnahme der Handorthese ist vorzugsweise als Unterarmmanschette ausgebildet, wobei die Unterarmaufnahme vorzugsweise den Unterarm zumindest bereichsweise und/oder abschnittsweise kontaktiert, insbesondere umschließt. Vorzugsweise liegt die Unterarmaufnahme zumindest bereichsweise und/oder abschnittsweise eng an dem Unterarm an. Vorzugsweise sind das Einstellglied und/oder der Korrekturindikator an der Unterarmaufnahme angeordnet und/oder an der Unterarmaufnahme befestigt. Insbesondere ist die Verstelleinrichtung, insbesondere das Einstellglied und/oder der Korrekturindikator, an der Oberseite der Unterarmaufnahme angeordnet und/oder befestigt.

Vorzugsweise verläuft das Übertragungsglied kontaktfrei und/oder kontaktbehaftet zur Unterarmaufnahme entlang der Längserstreckung der Unterarmaufnahme, insbesondere auf der Oberseite der Unterarmaufnahme. Vorzugsweise ist die Unterarmaufnahme derart an dem Unterarm fixiert, dass sie eine Gegenkraft für die mittels der Verstelleinrichtung erzeugte Korrekturkraft, insbesondere eine von der Verstelleinrichtung auf die Hand und/oder den zumindest einen Finger wirkende Zugkraft, erzeugt. Zum Erzeugen einer Gegenkraft der Unterarmaufnahme entgegen der mittels der Verstelleinrichtung erzeugten Korrekturkraft sollte sichergestellt sein, dass die Unterarmaufnahme derart an dem Unterarm fixiert ist, dass sie durch Wirkung der Korrekturkraft am Arm nicht verrutscht.

Es ist weiterhin eine erfindungsgemäße Handorthese bevorzugt, bei welcher die Unterarmaufnahme aus einem elastischen und/oder flexiblen Material ausgebildet ist und/oder ein elastisches und/oder flexibles Material umfasst. Alternativ oder zusätzlich ist die Unterarmaufnahme aus einem nicht elastischen und/oder nicht flexiblen Material ausgebildet und/oder umfasst ein-nicht elastisches und/oder nicht-flexibles Material. Das elastische und/oder dehnbare Material und/oder das nicht-elastische und/oder nicht-dehnbare Material kann das gleiche Material sein, aus welchem das Verbindungselement, die Fingeraufnahme und/oder das Übertragungsglied ausgebildet sind. Alternativ oder zusätzlich ist die Unterarmaufnahme aus einem atmungsaktiven Material ausgebildet und/oder umfasst ein atmungsaktives Material.

Vorzugsweise ist die Unterarmaufnahme zumindest bereichsweise und/oder zumindest abschnittsweise aus einem atmungsaktiven Material ausgebildet, insbesondere aus Mesh, sodass trotz einer eng an dem Unterarm anliegenden Unterarmaufnahme eine ausreichende Belüftung der darunterliegenden Haut sichergestellt werden kann. Insbesondere kann die Unterarmaufnahme gelöchert sein und/oder Lüftungsausnehmungen, beispielsweise Lüftungsschlitze, umfassen und/oder bereichsweise mit einer dünneren Materialstärke ausgeführt sein. Vorzugsweise ist die Unterarmaufnahme derart ausgebildet, dass es auch unter Krafteinwirkung, insbesondere unter Korrekturkrafteinwirkung, zu keiner Faltenbildung der Unterarmaufnahme kommt, sodass sichergestellt wird, dass die Unterarmaufnahme stets eng am Unterarm anliegt und nicht verrutschen kann, sodass ein Verrutschen der gesamten Handorthese und somit eine unbeabsichtigte Veränderung der Korrekturkraft und/oder der korrigierten Stellung vermieden werden kann. Die Unterarmaufnahme kann beispielsweise aus Mesh, Filz, Neopren, Leder, Stoff, und/oder Flachstrick ausgebildet sein und/oder Mesh, Filz, Neopren, Leder, Stoff, Gewebe und/oder Flachstrick zumindest bereichs- und/oder abschnittsweise umfassen. Die Unterarmaufnahme aus einem einzigen Material oder aus einem Materialmix aus mehreren unterschiedlichen Materialien ausgebildet sein.

Die der Erfindung zugrunde liegende Aufgabe wird weiterhin durch eine Orthesenanordnung der eingangs genannten Art gelöst, wobei der Stabilisierungskörper reversibel zerstörungsfrei lösbar an der Handorthese befestigbar ist, wobei der Stabilisierungskörper vorzugsweise dazu eingerichtet ist, die Hand und/oder zumindest einen Finger und/oder das Handgelenk in einer beabsichtigten Haltung ruhigzustellen. Vorzugsweise ist die Handorthese der erfindungsgemäßen Orthesenanordnung nach einer der vorstehenden Ausführungsformen ausgebildet. Hinsichtlich der Vorteile und Modifikationen der erfindungsgemäßen Orthesenanordnung wird folglich auf die Vorteile und Modifikationen der erfindungsgemäßen Handorthese verwiesen.

Vorzugsweise ist der Stabilisierungskörper als Nachlagerungsschiene zur Ruhigstellung der Hand und/oder des zumindest einen Fingers, beispielsweise für die Nachtruhe und/oder zur akuten Schmerzlinderung, ausgebildet, wobei der Stabilisierungskörper zur Ruhigstellung und/oder zur Fixierung der Hand in einer beabsichtigten Position verwendbar ist. Vorzugsweise ist der Stabilisierungskörper starr ausgebildet, insbesondere als starre Schiene. Vorzugsweise ist der Stabilisierungskörper wechselbar und/oder optional an der Handorthese befestigbar. Auf diese Weise kann der Stabilisierungskörper individuell bei Bedarf an der Handorthese befestigt und entfernt werden.

Vorzugsweise umfasst die Handorthese und/oder umfasst der Stabilisierungskörper ein oder mehrere Befestigungsglieder, beispielsweise Schlaufen und/oder Klettverschlüsse und/oder Klickverschlüsse, mittels welcher der Stabilisierungskörper an der Handorthese reversibel zerstörungsfrei wechselbar befestigt werden kann. Insbesondere kann der Stabilisierungskörper eine oder mehrere Fingerschlaufen und/oder eine oder mehrere Unterarmschlaufen umfassen, mittels welcher der Stabilisierungskörper an zumindest einem Finger und/oder an dem Unterarm durch Umschließen des zumindest einen Fingers und/oder des Unterarms mit der jeweiligen Schlaufe kraftschlüssig und/oder formschlüssig befestigbar ist. Die Schlaufen können beispielsweise aus Filz, Leder und/oder Gummi ausgebildet sein, insbesondere als Filzband, Lederband und/oder Gummiband. Die Schlaufen können zudem als Klettverschluss ausgebildet sein und/oder einen Klettverschluss umfassen. Zudem kann der Stabilisierungskörper dazu eingerichtet sein, den zumindest einen Finger, die Hand und/oder das Handgelenk sowie den Unterarm in einer beabsichtigten Haltung weich zu betten.

In einer bevorzugten Ausführungsform der erfindungsgemäßen Orthesenanordnung ist der Stabilisierungskörper aus einem formbaren und/oder aus einem nicht formbaren Material ausgebildet und/oder umfasst ein formbares und/oder nicht formbares Material. Alternativ oder zusätzlich kontaktiert der Stabilisierungskörper zumindest einen Finger und/oder eine Handfläche und/oder ein Handgelenk und/oder einen Unterarm zumindest bereichsweise. Alternativ oder zusätzlich ist der Stabilisierungskörper zumindest bereichsweise an den zumindest einen Finger und/oder die Handfläche und/oder das Handgelenk und/oder den Unterarm ergonomisch angepasst. Vorzugsweise ist der Stabilisierungskörper derart ausgebildet, dass der zumindest eine Finger und/oder die Hand in eine beabsichtigte Stellung verbringbar und/oder in der beabsichtigten Stellung fixierbar ist. Vorzugsweise ist der Stabilisierungskörper derart geformt, dass die Form des Stabilisierungskörpers mit einer gewünschten Stellung der Hand und/oder des zumindest einen Fingers korrespondiert, sodass die Hand und/oder der Finger durch Befestigen des Stabilisierungskörpers an der Handorthese in die gewünschte Stellung gebracht und/oder in der gewünschten Stellung fixiert wird.

Insbesondere ist der Stabilisierungskörper ergonomisch an die Form des Fingers und/oder der Hand und/oder des Unterarms, insbesondere an eine Neutralstellung, angepasst. Vorzugsweise ist der Stabilisierungskörper an der Unterseite des Unterarms und/oder an der Handinnenfläche und/oder an der Unterseite des zumindest einen Fingers und/oder des zumindest einen Handgelenks angeordnet. Vorzugsweise umfasst der Stabilisierungskörper zumindest abschnittsweise und/oder bereichsweise ein elastisch und/oder plastisch formbares Material, insbesondere Memoryschaum, Moosgummi und/oder Thermoplaste. Durch die Verwendung von formbaren Materialien ist der Stabilisierungskörper vorzugsweise individuell an die Form eines jeweiligen Fingers, einer jeweiligen Hand und/oder eines jeweiligen Unterarms, insbesondere an eine gewünschte Position und/oder Stellung, und somit individuell auf die Therapiebedürfnisse eines jeweiligen Patienten anpassbar. Auf diese Weise kann durch die Verwendung nachgiebiger Materialien eine Weichbettung erfolgen.

Die der Erfindung zugrunde liegende Aufgabe wird ferner durch ein Verfahren zum Einstellen der eingangs genannten Art gelöst, wobei im Rahmen des erfindungsgemäßen Verfahrens das Erzeugen einer auf die Fingeraufnahme wirkenden Korrekturkraft zum Verbringen des zumindest einen Fingers in eine korrigierte Stellung mittels einer Verstelleinrichtung der Handorthese erfolgt. Vorzugsweise wird im Rahmen des erfindungsgemäßen Verfahrens zum Einstellen eine Handorthese nach einer der vorstehenden Ausführungsformen eingestellt. Hinsichtlich der Vorteile und Modifikationen des erfindungsgemäßen Verfahrens zum Einstellen wird daher auf die Vorteile und Modifikationen der erfindungsgemäßen Handorthese verwiesen.

Nachfolgend werden bevorzugte Ausführungsformen der Erfindung unter Bezugnahme auf die beiliegenden Zeichnungen näher erläutert und beschrieben. Dabei zeigen:
- Fig. 1: eine erfindungsgemäße Handorthese in einer perspektivischen Darstellung von oben;
- Fig. 2: die Handorthese aus Fig. 1 in einer Draufsicht;
- Fig. 3: Fingeraufnahmen und ein Verbindungselement der Handorthese in einer Detailansicht von oben;
- Fig. 4: eine Verstelleinrichtung und eine Unterarmaufnahme der Handorthese in einer Detailansicht von oben; und
- Fig. 5: eine erfindungsgemäße Orthesenanordnung mit einer Handorthese und einem Stabilisierungskörper in einer perspektivischen Ansicht von oben.

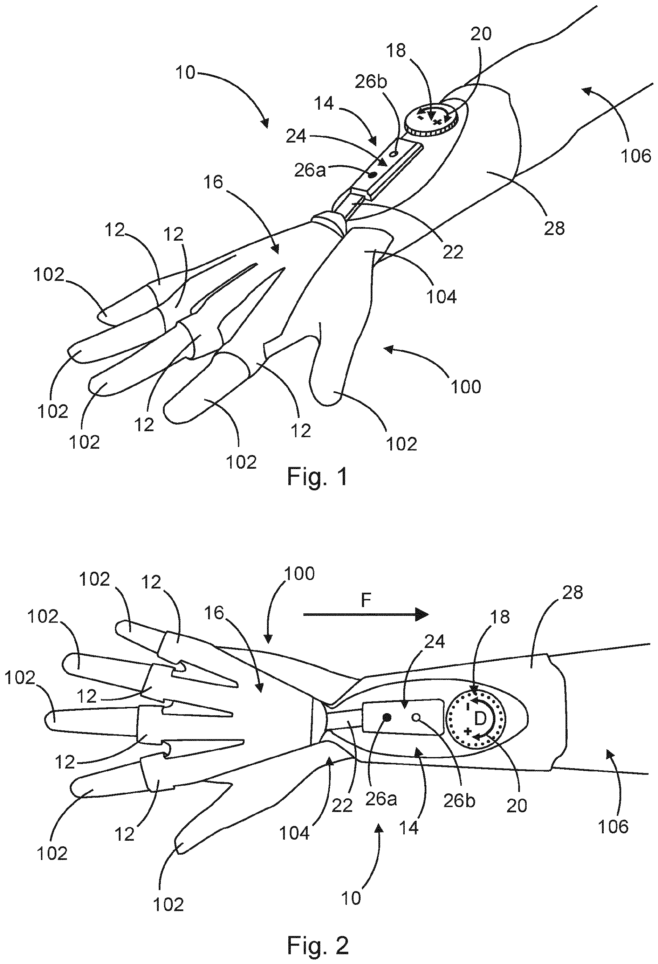

Die Fig. 1 und 2 zeigen eine erfindungsgemäße Handorthese 10, welche an einer Hand 100 und an einem Unterarm 106, insbesondere eines Patienten, angelegt ist. Dabei zeigt die Fig. 1 die angelegte Handorthese 10 in einer perspektivischen Ansicht und die Fig. 2 die angelegte Handorthese 10 in einer Draufsicht.

Die Handorthese 10 umfasst Fingeraufnahmen 12, welche Finger 102 der Hand 100 des Patienten aufnehmen, wobei die Fingeraufnahmen 12 ringförmig beziehungsweise schlaufenförmig ausgebildet sind und jede Fingeraufnahme 12 jeweils einen Finger 102 bereichsweise entlang des Umfangs des jeweiligen Fingers 102 umschließt und eng kontaktiert. Die dargestellte Handorthese 10 umfasst vier Fingeraufnahmen 12, sodass vier Finger 102 der Hand 100 von jeweils einer Fingeraufnahme 12 aufgenommen werden. Bei der dargestellten Ausführungsform der Handorthese 10 werden somit der kleine Finger, der Ringfinger, der Mittelfinger und der Zeigefinger jeweils von einer Fingeraufnahme 12 umschlossen. Es kann jedoch auch in anderen Ausführungsformen der Handorthese 10 vorgesehen sein, dass auch der Daumen von einer Fingeraufnahme 12 umschlossen wird und/oder dass einer oder mehrere der übrigen Finger 102 nicht von einer Fingeraufnahme 12 umschlossen werden.

Die Fingeraufnahmen 12 sind vorzugsweise aus einem elastischen und/oder dehnbaren Material, beispielsweise aus einem Elastomer, insbesondere Gummi, Neopren oder Silikon, ausgebildet. Die Fingeraufnahmen 12 sind dazu eingerichtet, eine Korrekturkraft, insbesondere eine Zugkraft, auf den jeweiligen umschlossenen Finger 102 zu übertragen, um den jeweiligen Finger 102 in eine beabsichtigte korrigierte Stellung zu verbringen. Zudem umfasst die Handorthese 10 ein Verbindungselement 16. Das Verbindungselement 16 ist vorzugsweise dazu eingerichtet, eine Korrekturkraft zum Verbringen der Finger 102 mittels der Fingeraufnahmen 12 auf die Fingeraufnahmen 12 zu übertragen, insbesondere durch Übertragen einer Zugkraft auf die Fingeraufnahmen 12. Das Verbindungselement 16 ist abschnittsweise gurtförmig und/oder bandförmig ausgebildet. An einem ersten Ende des Verbindungselements 16 ist das Verbindungselement 16 mit einer Verstelleinrichtung 14 der Handorthese 10, insbesondere mit einem Übertragungsglied 22 der Verstelleinrichtung 14, und/oder mit einer Unterarmaufnahme 28 der Handorthese 10 verbunden. Von diesem ersten Ende des Verbindungselements 16 erstreckt sich das Verbindungselement 16 entlang des Handrückens der Hand 100, wobei das Verbindungselement im Bereich des Handrückens der Hand 100 den Handrücken kontaktiert und eng an dem Handrücken anliegt.

Entlang der Erstreckung des Verbindungselements 16 zwischen dem ersten Ende und den Fingeraufnahmen 12 weist das Verbindungselement 16 abschnittsweise schlitzförmige Ausnehmungen auf, welche das Verbindungselement 16 bereichsweise in jeweils einer Fingeraufnahme 12 zugeordnete bandförmige Abschnitte unterteilt, wobei jeder dieser Abschnitte jeweils mit einer Fingeraufnahme 12 verbunden ist und/oder an jeweils einer Fingeraufnahme 12 befestigt ist. Die Abschnitte des Verbindungselements 16 verlaufen jeweils fluchtend und/oder parallel zu der Erstreckungsrichtung des jeweiligen Fingers 102, wobei die Abschnitte des Verbindungselements 16 jeweils den Handrücken der Hand 100 und/oder die Oberseite des jeweiligen Fingers 102 kontaktieren und/oder eng anliegen.

Das Verbindungselement 16 ist insbesondere aus einem elastischen und/oder dehnbaren Material, insbesondere aus einem Elastomer, wie Gummi, Neopren oder Silikon, ausgebildet. Vorzugsweise besteht das Verbindungselement 16 aus demselben Material wie die Fingeraufnahmen 12, wobei das Verbindungselement 16 und die Fingeraufnahmen 12 gemeinsam Bestandteil eines integralen Körpers sind. In anderen Ausführungsformen der Handorthese 10 können das Verbindungselement 16 und die Fingeraufnahmen 12 auch als getrennte Körper ausgebildet sein und/oder aus unterschiedlichen Materialien und/oder Materialzusammensetzungen bestehen.

Das Verbindungselement 16 ist dazu eingerichtet, eine mittels einer Verstelleinrichtung 14 erzeugte Korrekturkraft, mittels welcher die Hand 100, ein Handgelenk 104 der Hand 100, zumindest einer der Finger 102 und/oder der Unterarm 106 in eine korrigierte Stellung verbringbar ist, von der Verstelleinrichtung 14 auf die Fingeraufnahmen 12 zu übertragen. Die Korrekturkraft ist insbesondere eine in einer Korrekturkraftrichtung wirkende Zugkraft, wobei die Zugkraft mittels des Verbindungselements 16 auf die Fingeraufnahmen 12 zum Ziehen der Finger 102 in eine korrigierte Stellung übertragen wird. Die Verstelleinrichtung 14 ist an der Unterarmaufnahme 28 der Handorthese 10 angeordnet und/oder befestigt, wobei die Unterarmaufnahme 28 den Unterarm 106 entlang seines Umfangs bereichsweise aufnimmt, insbesondere umschließt und flächig eng kontaktiert. Dabei erstreckt sich die Unterarmaufnahme im Wesentlichen von dem Handgelenk 104 entlang eines Abschnitts des Unterarms 106 in Richtung des Ellenbogens.

Die Unterarmaufnahme 28 ist zumindest bereichsweise und/oder abschnittsweise aus einem atmungsaktiven Material, insbesondere aus einem Meshmaterial ausgebildet. Zudem kann die Unterarmaufnahme 28 zumindest bereichsweise und/oder abschnittsweise aus einem elastischen und/oder dehnbaren Material und/oder zumindest bereichsweise oder abschnittsweise aus einem nicht elastischen oder nicht dehnbaren Material ausgebildet sein. Beispielsweise kann die Unterarmaufnahme 28 zumindest bereichsweise und/oder abschnittsweise aus Mesh, Filz, Neopren, Leder, Stoff, Gewebe und/oder Flachstrick ausgebildet sein. Vorzugsweise ist die Unterarmaufnahme 28 derart ausgebildet und/oder derart an dem Unterarm 106 angeordnet, dass sie als Gegenkraft für die von der Verstelleinrichtung 14 erzeugten Korrekturkraft dient, sodass die erzeugte Korrekturkraft möglichst ohne Verluste auf die Fingeraufnahmen 12 übertragen werden kann, wobei die Unterarmaufnahme 28 unter Krafteinwirkung möglichst keine Falten bildet und/oder im Wesentlichen nicht entlang des Unterarms 106 verrutscht.

Die Verstelleinrichtung 14 der Handorthese 10, mittels welcher die Korrekturkraft erzeugbar ist, umfasst ein Einstellglied 18, ein Übertragungsglied 22 sowie einen Korrekturindikator 24. Das Einstellglied 18 der Verstelleinrichtung 14 ist als drehbares Einstellglied 18, insbesondere als Drehverschluss und/oder Drehratschenverschluss, besonders bevorzugt als drehbarer Schnellverschluss, ausgebildet. Durch Drehen des drehbaren Einstellglieds 18 kann die Korrekturkraft zum Verbringen der Hand 100 und/oder zumindest eines Fingers 102 individuell eingestellt werden. Durch Drehen des Einstellglieds 18 in der Drehrichtung D kann die Korrekturkraft insbesondere durch Drehen in eine erste Richtung erhöht und in eine entgegengesetzte zweite Richtung reduziert werden. Durch das Drehen an dem Einstellglied 18 wird die Drehbewegung des Einstellglieds 18 in eine Linearbewegung und/oder Lineardehnung eines Übertragungsglieds 22, insbesondere in der Korrekturkraftrichtung, der Verstelleinrichtung 14 umgesetzt.

Das Übertragungsglied 22 ist bandförmig ausgebildet, insbesondere als Gummiband, welches sich von dem Einstellglied 18 bis zum ersten Ende des Verbindungselements 16 erstreckt. Insbesondere erstreckt sich das Einstellglied 22 entlang der Längsrichtung des Unterarms 106 und/oder der Unterarmaufnahme 28 und verbindet das Einstellglied 18 mit dem Verbindungselement 16 und somit mit den Fingeraufnahmen 12. Durch Drehen des Einstellglieds 18 wird eine Zugkraft auf das Übertragungsglied 22 erzeugt, welche durch das Übertragungsglied 22 von dem Einstellglied 18 auf das Verbindungselement 16 und somit die Fingeraufnahmen 12 übertragen wird. Auf diese Weise führt eine Drehbewegung des Einstellglieds 18 zu einer Erhöhung und/oder einer Reduzierung der Zugkraft auf die Fingeraufnahmen 12 und somit auf die Finger 102.

Durch die Zugkraft können die Finger 102 und somit der Hand 100 in eine beabsichtigte korrigierte Stellung gebracht werden, sodass die Zugkraft einer Korrekturkraft zum Korrigieren der Hand- und/oder Fingerstellung entspricht. Somit kann durch Drehen des Einstellglieds 18 die Hand- und/oder Fingerstellung individuell eingestellt werden. Die Handorthese 10 ist durch diese individuell einstellbare und variable Korrekturkraft somit dazu eingerichtet, die Hand 100 und/oder die Finger 102 eines Patienten individuell auf eine Therapieform und/oder auf eine Erkrankung und/oder auf eine Verletzung so einzustellen, dass ungünstige Handpositionen, insbesondere eine Fallhand, vermieden werden und somit der Therapieerfolg erhöht wird und/oder eine Therapiedauer verkürzt wird.

Die Verstelleinrichtung 14 umfasst zudem einen Korrekturindikator 24, wobei sich das Übertragungsglied 22 durch den Korrekturindikator 24 hindurch erstreckt. Der Korrekturindikator 14 umfasst Signalanzeigen 26a, 26b, welche einem Benutzer der Handorthese 10, insbesondere dem Patienten, welcher die Handorthese 10 einstellt, die korrekte Einstellung der Handorthese 10, insbesondere eine beabsichtigte Korrekturkraft und/oder das Vorhandensein einer beabsichtigten korrigierten Stellung der Hand 100 und/oder der Finger 102, anzeigen. Alternativ oder zusätzlich zeigen die Signalanzeigen 26a, 26b an, sobald sich die Korrekturkraft aus einem gewünschten Korrekturkraftbereich herausbewegt und/oder sobald die Hand 100 und/oder die Finger 102 drohen, sich aus einer beabsichtigten Korrekturstellung herauszubewegen. Insbesondere kann über die Signalanzeigen 26a, 26b abgelesen werden, ob sich die Hand 100 in einer für eine Therapie ungünstigen Fallhandposition befindet und/oder droht in eine Fallhandposition überzugehen.

Die Signalanzeigen 26a, 26b sind in der dargestellten Ausführungsform der Handorthese 10 als farbliche Signalanzeigen, insbesondere als runde farbliche Signalbereiche ausgebildet, wobei die Signalanzeige 26a durch eine rote Signalfarbe anzeigt, dass sich die eingestellte Korrekturkraft und/oder die daraus resultierende korrigierte Stellung aktuell in einem nicht tolerierbaren Bereich befinden, und wobei die Signalanzeige 26b durch eine grüne Signalfarbe anzeigt, dass sich die eingestellte Korrekturkraft und die daraus resultierende korrigierte Stellung in einem gewünschten Bereich befindet. Durch den Korrekturindikator 24 kann somit das korrekte Einstellen der Handorthese 10 erleichtert werden und schnell und einfach erkannt werden, sobald die Einstellung der Handorthese 10 fehlerhaft ist, sodass die Einstellung der Handorthese 10 in einem solchen Fall schnell korrigiert werden kann.

Die angezeigte grüne oder rote Signalfarbe über die Signalanzeigen 26a, 26b wird insbesondere durch die von dem Übertragungsglied 22 übertragene Korrekturkraft, insbesondere die übertragene Zugkraft, beeinflusst, da sich das Übertragungsglied 22 durch den Korrekturindikator 24 hindurch erstreckt und die auf das Übertragungsglied 22 wirkende Korrekturkraft und/oder Zugkraft abgeleitet werden kann, ob die auf die Finger 102 und/oder Hand 100 wirkende Korrekturkraft korrekt ist.

Das Übertragungsglied 22, welches das Einstellglied 18 mit dem Verbindungselement 16 verbindet, kann aus dem gleichen Material ausgebildet sein, wie das Verbindungselement 16 und/oder die Fingeraufnahmen 12. Das Übertragungsglied 22, das Verbindungselement 16 und/oder die Handaufnahmen 12 können einen gemeinsamen integralen Körper bilden. Das Übertragungsglied 22 kann zudem als separater Körper ausgebildet sein, welcher aus einem elastischen und/oder dehnbaren Material und/oder aus einem nicht elastischen und/oder nicht dehnbaren Material ausgebildet ist.

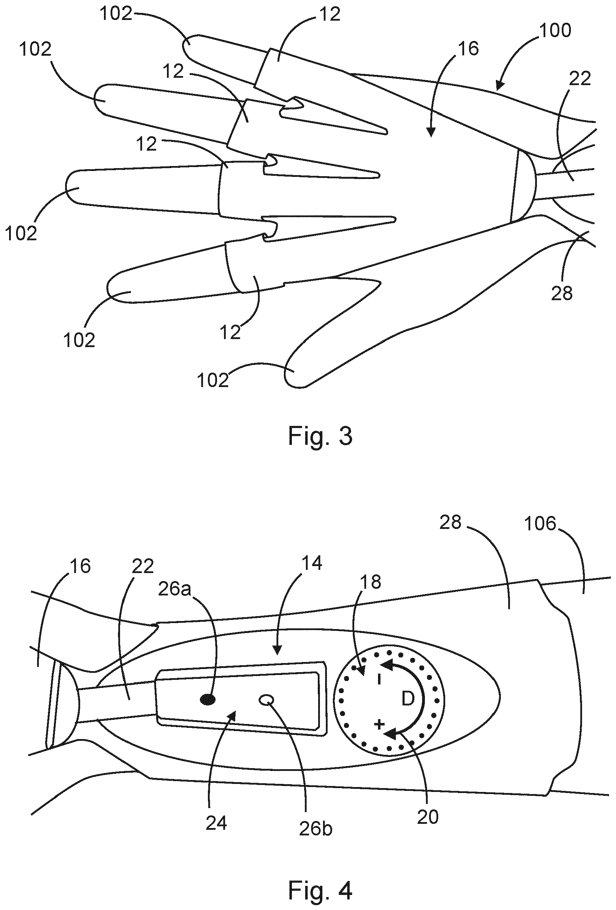

Die Fig. 3 zeigt eine Detailaufnahme der Fingeraufnahmen 12 sowie das Verbindungselement 16 der Handorthese 10 in einer Draufsicht. Das Verbindungselement 16 ist dazu eingerichtet, die Korrekturkraft, insbesondere die Zugkraft, welche von dem Übertragungsglied 22 von der Verstelleinrichtung 14 übertragen wird, entlang des Handrückens der Hand 100 auf die Fingeraufnahmen 12, welche jeweils einen Finger 102 umschließen, zu übertragen. Das Verbindungselement 16 ist an seinem rechten ersten Ende mit dem Übertragungsglied 22 verbunden und/oder an dem Übertragungsglied 22 befestigt. Entlang des Handrückens der Hand 100 ist das Verbindungselement 16 an dem den Fingern 102 zugewandten Bereich des Verbindungselements 16 in seiner Längserstreckung, insbesondere in Parallelrichtung zu der Längserstreckung der Finger 102, so eingeschlitzt, dass vier bandförmige Bereiche des Verbindungselements 16, welche jeweils in Richtung eines der Finger 102 der Hand 100 verlaufen, ausgebildet werden, wobei am Ende jedes bandförmigen Abschnitts des Verbindungselements 16 eine Fingeraufnahme 12 zum Umschließen jeweils eines Fingers 102 angeordnet ist.

In der dargestellten Ausführungsform bilden das Verbindungselement 16 und die Fingeraufnahmen 12 einen einstückigen integralen Körper und sind aus demselben elastischen und dehnbaren Material ausgebildet. Auf diese Weise wird eine Korrekturkraft von der Verstelleinrichtung 14 über das Übertragungsglied 22 auf das Verbindungselement 16 übertragen und von dem Verbindungselement 16 wiederum auf die Fingeraufnahmen 12 übertragen, sodass eine durch die Verstelleinrichtung 14 erzeugte Korrekturkraft, insbesondere eine Zugkraft, in der Korrekturkraftrichtung F derart auf die Finger 102 der Hand 100 wirkt, dass die Finger 102 und somit die gesamte Hand 100 durch die Korrekturkraft in eine beabsichtigte korrigierte Stellung verbringbar ist.

Die Fig. 4 zeigt eine Detailansicht der Unterarmaufnahme 28 sowie der darauf angeordneten Verstelleinrichtung 14 der Handorthese 10 in einer Draufsicht. Die Verstelleinrichtung 14 ist auf der Oberseite der Unterarmaufnahme 28, welche den oberen Bereich des Unterarms 106 kontaktiert, angeordnet. Das Einstellglied 20, welches als drehbares Einstellglied 20 ausgebildet ist, kann in der Drehrichtung D im Uhrzeigersinn und/oder gegen den Uhrzeigersinn gedreht werden. Das Einstellglied 18 umfasst eine Beschriftung 20, welche die Drehrichtung D anzeigt und durch ein Pluszeichen und ein Minuszeichen anzeigt, in welcher Drehrichtung D die durch das Einstellglied 18 erzeugte Korrekturkraft, insbesondere die auf das Übertragungsglied 22 ausgeübte Zugkraft, erhöht oder reduziert wird. Das Einstellglied 18 umfasst außerdem entlang seines Außenumfangs eine Riffelung, welche in der dargestellten Ausführungsform zusätzlich eine Skala mit Teilstrichen aufweist, durch welche das Drehen des Einstellglieds 18 erleichtert wird.

Wird an dem Einstellglied 18 im Uhrzeigersinn in der Drehrichtung D gedreht, wird die Drehbewegung des Einstellglieds 18 in eine Linearbewegung des Übertragungsglieds 22 umgesetzt, wobei das Einstellglied 18 durch die Drehbewegung an dem Einstellglied 22 zieht. Durch die Zugkraft auf das Übertragungsglied 22 wird an dem mit dem Übertragungsglied 22 verbundenen Verbindungselement 16 gezogen. Auf diese Weise wird die durch die Verstelleinrichtung 14 erzeugte Korrekturkraft in Form einer Zugkraft über das Übertragungsglied 22 und das Verbindungselement 16 auf die Fingeraufnahmen 12 der Handorthese 10 übertragen.

Das bandförmige und aus einem elastischen Material ausgebildete Übertragungsglied 22 erstreckt sich entlang der Längserstreckung der Unterarmaufnahme 28 von dem Einstellglied 18 bis hin zum Verbindungselement 16, wobei das Übertragungsglied 22 entlang seiner Längserstreckung durch den Korrekturindikator 24 verläuft. Durch die auf das Übertragungsglied 22 wirkende Zugkraft und die daraus resultierende Dehnung des Übertragungsglieds 22 und/oder die daraus resultierende Linearbewegung und/oder Lineardehnung des Übertragungsglieds 22 führt zu einer Zustandsänderung des Korrekturindikators 24, insbesondere zu einer Zustandsänderung und/oder einer Signaländerung der von Signalanzeigen 26a, 26b angezeigten Signale zum Erfassen und/oder Ablesen einer aktuell eingestellten Korrekturkraft, einer aktuell vorliegenden und aus der aktuellen Korrekturkraft resultierenden korrigierten Stellung und/oder eine aktuelle Veränderung der Korrekturkraft und/oder eine aktuelle aus der Veränderung der Korrekturkraft resultierende Veränderung der korrigierten Stellung.

Die Signalanzeigen 26a, 26b des Korrekturindikators 24 zeigen mittels eines farbigen Signals den aktuellen Zustand der Handorthese 10 an. Die Signalanzeige 26a zeigt durch eine rote Farbe an, wenn die eingestellte Korrekturkraft außerhalb eines beabsichtigten Korrekturkraftbereichs liegt. Zudem kann das Anzeigen einer roten Farbe durch die Signalanzeige 26a anzeigen, wenn sich die Hand 100 und/oder die Finger 102 aktuell nicht in einer beabsichtigten korrigierten Stellung befinden, beispielsweise in einer Fallhandposition. Die Signalanzeige 26b kann mittels einer grünen Farbe anzeigen, wenn sich die eingestellte Korrekturkraft aktuell in einem gewünschten Korrekturkraftbereich befindet und/oder dass die Hand 100 und/oder die Finger 102 aktuell in einer beabsichtigten korrigierten Stellung eingestellt sind. In anderen Ausführungsformen der Handorthese 10 kann das Anzeigen der Korrekturkraft und/oder der beabsichtigten Stellung auch durch andersartig ausgebildete Signalanzeigen angezeigt werden. Es ist denkbar, dass der Korrekturindikator 24 in anderen Ausführungsformen beispielsweise einen Lautsprecher zum Ausgeben von Signaltönen und/oder ein Display zum Anzeigen von Zahlenwerten, beispielsweise zum Anzeigen einer aktuell eingestellten Korrekturkraft, und/oder Signallämpchen, welche durch Aufleuchten, insbesondere in unterschiedlichen Farben, den aktuellen Status der Handorthese 10 anzeigen.

Die Verstelleinrichtung 14 ist vorzugsweise derart ausgebildet, dass eine gewünschte erzeugte Korrekturkraft und/oder eine aus der Korrekturkraft resultierende korrigierte Stellung bei einem gewünschten Wert verriegelt und/der fixiert wird. Dazu kann beispielsweise das Einstellglied 18 dazu eingerichtet sein, in einer aktuellen Position und einer daraus resultierenden Korrekturkraft zu verbleiben, solange keine äußere Kraft auf das Einstellglied 18, beispielsweise durch manuelles Drehen des Einstellglieds 18, wirkt. Dazu kann das Einstellglied 18 eine interne Mechanik, beispielsweise eine Verzahnung aufweisen, welche außerdem zusätzlich durch manuelles Ver- und Entriegeln des Einstellglieds, beispielsweise durch Herausziehen oder Hineindrücken des Einstellglieds senkrecht zur Drehrichtung, verriegelt und/oder entriegelt werden kann, um eine aktuell eingestellte Korrekturkraft und/oder eine aktuell eingestellte korrigierte Stellung zu fixieren und/oder wieder zu lösen.

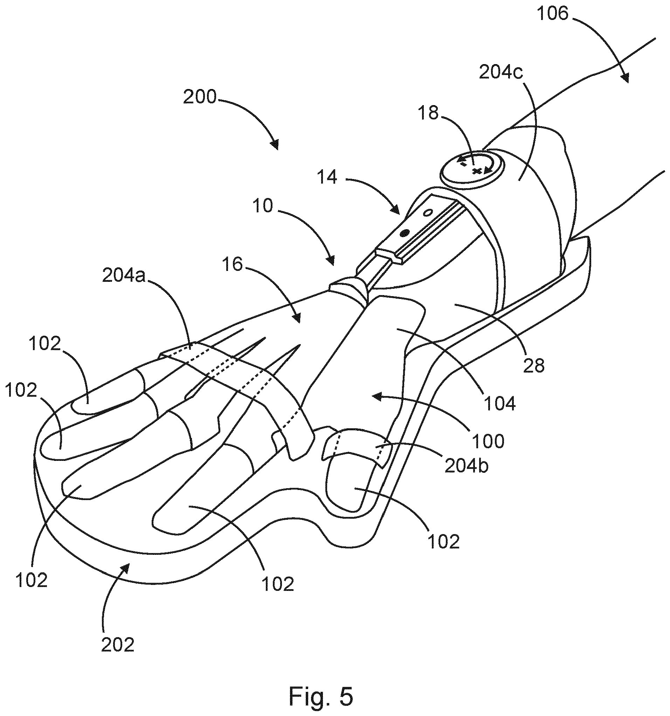

Die Fig. 5 zeigt eine erfindungsgemäße Orthesenanordnung 200, welche eine Handorthese 10, insbesondere eine Handorthese 10 wie sie in den Fig. 1 bis 4 dargestellt ist, umfasst und welche einen Stabilisierungskörper 202 umfasst. Die Orthesenanordnung 200 mit der Handorthese 10 und dem Stabilisierungskörper 202 ist in der Fig. 5 in einer perspektivischen Ansicht von oben dargestellt.

Der Stabilisierungskörper 202 ist als Auflage für die Hand 100, die Finger 102 sowie eines Abschnitts des Unterarms 106, insbesondere als schienenförmige Auflage, besonders bevorzugt als Nachlagerungsschiene, ausgebildet. Der Stabilisierungskörper 202 erstreckt sich von dem zum Ellenbogen zugewandten Ende der Unterarmaufnahme 28 der Handorthese 10 bis hin zu den Fingerspitzen der Finger 102, wobei der Stabilisierungskörper 202 an der Unterseite des Unterarms 106 sowie der Hand 100 angeordnet ist. Insbesondere kontaktiert der Stabilisierungskörper 202 die Innenfläche der Hand 100, die innenseitigen Flächen der Finger 102 sowie die Innenseite des Handgelenks 104 und des Unterarms 106 zumindest bereichsweise. Die Hand 100 sowie der Unterarm 106, die Finger 102 und das Handgelenk 104 liegen auf dem Stabilisierungskörper 202 flächig auf.

Der Stabilisierungskörper 202 ist über Befestigungsglieder 204a-204c an der Handorthese 10 und/oder an der Hand 100 und/oder an dem Unterarm 106 befestigt. Die Befestigungsglieder 204a-204c sind schlaufenförmig ausgebildet. Das Befestigungsglied 204a erstreckt sich in Querrichtung zur Längserstreckung der Hand 100 über den Handrücken der Hand 100 sowie über das Verbindungselement 16, insbesondere über die bandförmigen Abschnitte des Verbindungselements 16, hinweg und kontaktiert das Verbindungselement 16 und/oder den Handrücken der Hand 100 zumindest bereichsweise. Insbesondere wird über das Befestigungsglied 204a eine Druckkraft von oben auf das Verbindungselement 16 und auf die Hand 100 ausgeübt, sodass die Hand 100 mit der Handinnenfläche auf den Stabilisierungskörper 202 zumindest leicht angepresst wird. Das Befestigungsglied 204b erstreckt sich in Querrichtung zur Längserstreckung des Daumens der Hand 100, sodass der Daumen ebenfalls mit einer zumindest leichten Druckkraft auf den Stabilisierungskörper 202 angedrückt wird. Das Befestigungsglied 204c erstreckt sich vorzugsweise in Querrichtung zur Längserstreckung des Unterarms 106 und kontaktiert insbesondere die Unterarmaufnahme 28 zumindest bereichsweise und die Verstelleinrichtung 14 zumindest bereichsweise. Das Befestigungsglied 204c ist vorzugsweise breiter ausgeführt als die Befestigungsglieder 204a und 204b, wobei sich das Befestigungsglied 204 zumindest abschnittsweise in Umfangsrichtung um die Unterarmaufnahme 28 erstreckt und zumindest eine leichte Druckkraft auf den Unterarm in Richtung des Stabilisierungskörpers 202 auswirkt, sodass der Unterarm 106 mit seiner Unterseite an den Stabilisierungskörper 202 angedrückt wird.

Die Befestigungsglieder 204a-204c sind jeweils als bandförmige Schlaufen ausgebildet, wobei die Befestigungsglieder 204a-204c aus einem dehnbaren und/oder elastischen und/oder aus einem nicht-dehnbaren und/oder nicht-elastischen Material ausgebildet sein können. Die Befestigungsglieder 204a-204c umfassen beispielsweise Filz, Leder und/oder Gummi. Vorzugsweise sind die Befestigungsglieder 204a-204c als Filzband, Lederband und/oder Gummiband ausgebildet, wobei das Material insbesondere atmungsaktiv ausgebildet ist, beispielsweise indem in den Befestigungsgliedern 204a-204c schlitzförmige und/oder kreisförmige Ausnehmungen angeordnet sein können, durch welche die Befestigungsglieder 204a-204c luftdurchlässig werden. Die Befestigungsglieder 204a-204c können zudem aus einem Meshmaterial bestehen und/oder zumindest abschnittsweise Meshmaterial umfassen.

Der Stabilisierungskörper 202 ist somit reversibel zerstörungsfrei lösbar über die Befestigungsglieder 204a-204c mit der Handorthese 10 verbunden, sodass der Stabilisierungskörper 202 als abnehmbare und/oder wechselbare Nachlagerungsschiene ausgebildet ist. Der Stabilisierungskörper 202 ist insbesondere dazu eingerichtet, die Finger 102, die Hand 100, das Handgelenk 104 und/oder den Unterarm 106, insbesondere in Kombination mit der Handorthese 10 zur Unterstützung der Handorthese 10, in einer beabsichtigten Haltung ruhigzustellen und/oder zu stabilisieren. Beispielsweise kann der Stabilisierungskörper 202 eingesetzt werden, um zu Beginn einer Therapie durch zusätzliche Ruhigstellung Schmerzen zu lindern und/oder für eine Weichbettung zu sorgen.

Der Stabilisierungskörper 202 ist zumindest bereichsweise aus formbaren und/oder nachgiebigen Materialien, beispielsweise Memoryschaum, Moosgummi und/oder Thermoplasten, ausgebildet, sodass der Stabilisierungskörper 202 ergonomisch an die Hand 100, die Finger 102 und/oder den Unterarm 106 anpassbar ist und/oder sich beim Anlegen des Stabilisierungskörpers 202 durch Verformung des Materials des Stabilisierungskörpers 202 automatisch an die Konturen der Hand 100, der Finger 102 und/oder des Unterarms 106 anpasst. Der Stabilisierungskörper 202 kann sich somit an die Konturen anschmiegen. Auf diese Weise kann die Hand 100 mit den Fingern 102 und/oder mit dem Unterarm 106 in eine gewünschte Stellung, insbesondere in eine Neutralstellung gebracht werden, um die Therapie mittels der Handorthese 10 zu unterstützen.

### Bezugszeichen

- 10: Handorthese
- 12: Fingeraufnahme
- 14: Verstelleinrichtung
- 16: Verbindungselement
- 18: Einstellglied
- 20: Beschriftung
- 22: Übertragungsglied
- 24: Korrekturindikator
- 26a, 26b: Signalanzeigen
- 28: Unterarmaufnahme

- 100: Hand
- 102: Finger
- 104: Handgelenk
- 106: Unterarm

- 200: Orthesenanordnung
- 202: Stabilisierungskörper
- 204a-204c: Befestigungsglied

- D: Drehrichtung
- F: Korrekturkraftrichtung

## Patentansprüche

1. Handorthese (10), mit
- zumindest einer Fingeraufnahme (12), welche dazu eingerichtet ist, zumindest einen Finger (102) einer Hand (100) zumindest bereichsweise aufzunehmen, insbesondere zu umschließen;
**gekennzeichnet durch** eine Verstelleinrichtung (14), welche dazu eingerichtet ist, eine auf die Fingeraufnahme (12) wirkende Korrekturkraft zu erzeugen, durch welche der zumindest eine Finger (102) in eine korrigierte Stellung verbringbar ist.

2. Handorthese (10) nach Anspruch 1,
**gekennzeichnet durch** zumindest ein Verbindungselement (16), welches die Verstelleinrichtung (14) mit der zumindest einen Fingeraufnahme (12) verbindet und dazu eingerichtet ist, die mittels der Verstelleinrichtung (14) erzeugte Korrekturkraft auf die Fingeraufnahme (12) zu übertragen.

3. Handorthese (10) nach Anspruch 2,
**dadurch gekennzeichnet, dass** das Verbindungselement (16)
- aus einem elastischen und/oder flexiblen Material ausgebildet ist und/oder ein elastisches und/oder flexibles Material umfasst, und/oder
- aus einem nicht-elastischen und/oder nicht-flexiblen Material ausgebildet ist und/oder ein nicht-elastisches und/oder nicht-flexibles Material umfasst,
wobei das Verbindungselement (16) und die zumindest eine Fingeraufnahme (12) vorzugsweise Bestandteil eines gemeinsamen integralen Körpers sind.

4. Handorthese (10) nach einem der vorstehenden Ansprüche,
**dadurch gekennzeichnet, dass** die Verstelleinrichtung (14) als mechanische Verstelleinrichtung (14) ausgebildet ist, wobei
- die Korrekturkraft vorzugsweise mittels eines Einstellglieds (18) der Verstelleinrichtung (14) erzeugbar und/oder verstellbar ist, und/oder
- das Einstellglied (18) der Verstelleinrichtung (14) zum Fixieren einer beabsichtigten Korrekturkraft in einer gewünschten Stellung festsetzbar ist.

5. Handorthese (10) nach Anspruch 4,
**dadurch gekennzeichnet, dass** das Einstellglied (18) als drehbares Einstellglied (18), insbesondere als Drehverschluss, ausgebildet ist.

6. Handorthese (10) nach einem der Ansprüche 4 oder 5,
**dadurch gekennzeichnet, dass** die Verstelleinrichtung (14) ein Übertragungsglied (22) zum Übertragen der Korrekturkraft von dem Einstellglied (18) der Verstelleinrichtung (14) auf die Fingeraufnahme (12), insbesondere über das Verbindungselement (16), umfasst.

7. Handorthese (10) nach Anspruch 6,
**dadurch gekennzeichnet, dass** das Übertragungsglied (22)
- aus einem elastischen und/oder flexiblen Material ausgebildet ist und/oder ein elastisches und/oder flexibles Material umfasst, und/oder
- aus einem nicht-elastischen und/oder nicht-flexiblen Material ausgebildet ist und/oder ein nicht-elastisches und/oder nicht-flexibles Material umfasst,
wobei das Übertragungsglied (22) vorzugsweise bandförmig ausgebildet und/oder mit dem Verbindungselement (16) verbunden ist.

8. Handorthese (10) nach einem der vorstehenden Ansprüche, **gekennzeichnet durch** einen Korrekturindikator (24), über welchen erfassbar ist, ob die mittels der Verstelleinrichtung (14) eingestellte Korrekturkraft und/oder die daraus resultierende korrigierte Stellung des zumindest einen Fingers (102) in einem beabsichtigten Korrekturbereich liegen.

9. Handorthese (10) nach Anspruch 8,
**dadurch gekennzeichnet, dass** der Korrekturindikator (24) dazu eingerichtet ist, ein die eingestellte Korrekturkraft und/oder die daraus resultierende korrigierte Stellung betreffendes optisches und/oder haptisches und/oder akustisches Signal zu erzeugen, wobei der Korrekturindikator (24) vorzugsweise Bestandteil der Verstelleinrichtung (14) ist.

10. Handorthese (10) nach einem der vorstehenden Ansprüche,
**gekennzeichnet durch** eine Unterarmaufnahme (28), welche dazu eingerichtet ist, einen Unterarm (106) zumindest bereichsweise aufzunehmen, insbesondere zu umschließen, wobei die Verstelleinrichtung (14) vorzugsweise an der Unterarmaufnahme (28) angeordnet ist und/oder die Unterarmaufnahme (28) mit der Fingeraufnahme (12) und/oder mit dem Verbindungselement (16) verbunden ist.

11. Handorthese (10) nach Anspruch 10,
**dadurch gekennzeichnet, dass** das die Unterarmaufnahme (28)
- aus einem elastischen und/oder flexiblen Material ausgebildet ist und/oder ein elastisches und/oder flexibles Material umfasst, und/oder
- aus einem nicht-elastischen und/oder nicht-flexiblen Material ausgebildet ist und/oder ein nicht-elastisches und/oder nicht-flexibles Material umfasst, und/oder
- aus einem atmungsaktiven Material ausgebildet ist und/oder ein atmungsaktives Material umfasst.

12. Orthesenanordnung (200), mit
- einer Handorthese (10), insbesondere einer Handorthese (10) nach einem der vorstehenden Ansprüche, und
- einem Stabilisierungskörper (202), welcher an der Handorthese (10) anordenbar ist,
**dadurch gekennzeichnet, dass** der Stabilisierungskörper (202) reversibel zerstörungsfrei lösbar an der Handorthese (10) befestigbar ist, wobei der Stabilisierungskörper (202) vorzugsweise dazu eingerichtet ist, die Hand (100) und/oder zumindest einen Finger (102) und/oder das Handgelenk (104) in einer beabsichtigten Haltung ruhigzustellen.

13. Orthesenanordnung (200) nach Anspruch 12,
**dadurch gekennzeichnet, dass** der Stabilisierungskörper (202)
- aus einem formbaren und/oder aus einem nicht-formbaren Material ausgebildet ist und/oder ein formbares und/oder nicht-formbares Material umfasst, und/oder
- zumindest einen Finger (102) und/oder eine Handfläche und/oder ein Handgelenk (104) und/oder einen Unterarm (106) zumindest bereichsweise kontaktiert, und/oder
- zumindest bereichsweise an den zumindest einen Finger (102) und/oder die Handfläche und/oder das Handgelenk (104) und/oder den Unterarm (106) ergonomisch angepasst ist,
wobei der Stabilisierungskörper (202) vorzugsweise derart ausgebildet ist, dass der zumindest eine Finger (102) und/oder die Hand (100) in eine beabsichtigte Stellung verbringbar und/oder in der beabsichtigten Stellung fixierbar ist.

14. Verfahren zum Einstellen einer Handorthese (10), insbesondere einer Handorthese (10) nach einem der Ansprüche 1 bis 11, mit dem Schritt:
- zumindest bereichsweises Aufnehmen, insbesondere Umschließen, zumindest eines Fingers (102) einer Hand (100) mittels zumindest einer Fingeraufnahme (12) der Handorthese (10),
**gekennzeichnet durch** den Schritt:
- Erzeugen einer auf die Fingeraufnahme (12) wirkenden Korrekturkraft zum Verbringen des zumindest einen Fingers (102) in eine korrigierte Stellung mittels einer Verstelleinrichtung (14) der Handorthese (10).
